# EUROPEAN PATENT SPECIFICATION

(11) **EP 2 995 272 B1**
(45) Date of publication and mention of the grant of the patent: **18.10.2017**
(21) Application number: 13883985.7
(22) Date of filing: 07.05.2013
(51) Int. Cl.: A61B 18/20, A61M 37/00, A61N 5/06

(54) **IMPROVED METHOD FOR DEPILATION BY PHOTOTHERMOLYSIS WITH MELANIN**
VERBESSERTES VERFAHREN ZUR DEPILATION DURCH PHOTOTHERMOLYSE MIT MELANIN
PROCÉDÉ AMÉLIORÉ D'ÉPILATION PAR PHOTOTHERMOLYSE AVEC DE LA MÉLANINE

(43) Date of publication of application: 16.03.2016
(73) Proprietor: Dermopartners, S.L., 46138 Rafelbunyol - Valencia (ES)
(72) Inventor: SERRANO SANMIGUEL, Gabriel, E-46138 Rafelbunyol - Valencia (ES); SERRANO NUÑEZ, Juan Manuel, E-46138 Rafelbunyol - Valencia (ES); SERRANO NUÑEZ, Gabriel, E-46138 Rafelbunyol - Valencia (ES)
(74) Representative: Urizar Anasagasti, Jesus Maria
(86) International application number: PCT/ES2013/070088
(87) International publication number: WO 2014/181002

(56) References cited:
- EP-A1- 1 764 129
- EP-A1- 2 286 760
- WO-A1-00/40162
- WO-A1-97/38638
- WO-A1-97/38638
- WO-A1-2009/073017
- WO-A1-2009/073017
- US-A1- 2009 118 698
- SAND M ET AL.: 'A Randomized, Controlled, Double-Blind Study Evaluating Melanin-Encapsulated Liposomes as a Chromophore for Laser Hair Removal of Blond, White, and Gray Hair.' ANNALS OF PLASTIC SURGERY. vol. 58, no. 5, 2007, pages 551 - 554, XP008180839

## Description

### OBJECT OF THE INVENTION

The proposed invention relates to a new improved melanin-enhanced photothermolysis hair removal method for white, blond or gray hair applicable to the beauty and photoepilation salon industry.

### BACKGROUND OF THE INVENTION

The fundamental principle of laser hair removal is selective photothermolysis (SPTL), the coincidence of a specific wavelength of light and a duration of impulses such as to obtain an optimum effect on a chosen tissue with a minimum effect on the surrounding tissue. Lasers can cause localized damage on selectively heating dark target matter, melanin, in the area that causes hair growth, the follicle, while not heating the rest of the skin. The light is absorbed by the dark objects so that the laser energy may be absorbed by dark skin matter, but with much greater speed and intensity. This dark target matter, or chromophore, may be natural or may be introduced artificially.

Melanin is considered to be the main chromophore for laser hair removal. Melanin occurs naturally in the skin and gives skin and hair their color. There are two types of melanin in hair. Eumelanin gives hair its chestnut or brown color, while pheomelanin gives hair its blond or red color. Due to the selective absorption of laser light photons, only black or chestnut hair can be removed. Laser works best with thick brown hair. Fair skin and brown hair are an ideal combination, being the most effective and producing the best results, but the new lasers are now capable of targeting dark brown hair with some success in dark-skinned patients.

The new lasers can target hair with little or no melanin by applying liposome-encapsulated artificial melanin solutions onto the skin, which is only accumulated in the hair and its follicle. In this manner, even patients with lighter-colored hair can remove unwanted hair using laser or IPL (Intense Pulsed Light) devices. The idea of selective staining of follicles of blond, white and gray hair could be an excellent approach to permanent laser hair removal.

Liposomal melanin lotions have been used in recent years with conflicting results.

In this connection, mention should be made of the pre-existence of patent US5766214 (Thomas L. Mehl and Nardo Zaias), where reference is made to the foregoing, through the topical application of liposome-encapsulated melanin to darken the hair follicles before subjecting the skin to photoepilation.

The main drawback of this method is that the patient must apply the melanin preparation six to eight times a day, two to five weeks prior to photoepilation, in order to allow absorption by the hair follicles. Successful hair removal was proportional to the amount of solution applied, i.e. the application of greater amounts of product leads to greater hair removal but with a higher cost.

Recently, the use of dermal puncture has been proposed as a new physical strategy for increasing the transdermal application of drugs. Since 1995, this technique has been used to achieve percutaneous collagen induction aimed at reducing skin imperfections. To date, dermal puncture has been proposed mainly as an effective method for treating scars and wrinkles, and is carried out by rolling a special device over the skin which comprises a rotating body having a variable number of microneedles.

These types of devices are disclosed in patents WO0249711 (Horst LIEBL) and EP1764129 (Horst LIEBL).

### DESCRIPTION OF THE INVENTION

The improved melanin-enhanced photothermolysis hair removal method proposed by the invention consists of an improved method of applying artificial melanin for the permanent removal of white, gray and blond hair based on the use of light energy that is absorbed by topically applied artificial melanin granules, resulting in death of the hair-producing tissue.

This method reduces the application time for introducing artificial melanin in the hair follicle and the amount of product required to induce hair removal.

Liposome-encapsulated artificial melanin penetrates in greater amounts and more quickly through the skin using a roller device having microneedles.

After the liposomal melanin solution is applied onto the skin, the roller is made to roll over the skin. In this case the needles penetrate the skin, opening micro-channels wherethrough melanin penetrates through the epidermis until reaching the hair follicle.

In such cases, this artificial melanin in the hair follicle is the chromophore for the selective photothermolysis of unwanted non-pigmented hair. Since the channels in the skin remain open for a long time, additional melanin can penetrate through the skin. Optionally, this method can be enhanced by means of a massage or similar measures.

Depending on the frequency with which the device moves over the skin, the number of channels opened therein may be specifically controlled, in addition to the degree to which the melanin can penetrate through the treated skin. Since the opened channels close again after a few minutes, damage to the epidermis is minimal. Therefore, the initially mentioned damage related to the removal of the epidermis does not occur in the case of the invention. In any case, light reddening of the skin is observed which, however, subsides completely in a few days, in most cases in one or two days. Therefore, the skin treatment practically has no side-effects and, in general, is completely painless.

Another advantage consists of the fact that the time required for the product to penetrate through the skin (hair follicle) and also the amount of product used may be considerably reduced, since the penetration of the ingredient through the lower skin layers is highly effective. All this simplifies and cheapens the method.

### DESCRIPTION OF THE DRAWINGS

In order to supplement this specification and aid understanding thereof, the following drawings have been included:

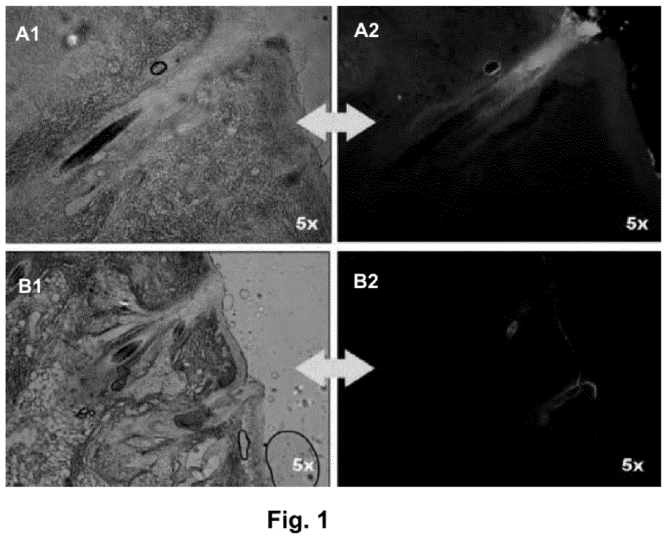

FIGURE 1. Shows images of the skin marked with a fluorescent dye to detect the location of liposome-encapsulated melanin for laser removal of white hair. A1 and B1 correspond to samples without fluorescence excitation. A2 and B2 show the exact location of the melanin inside the hair, due to which it is a good target for light absorption to achieve removal thereof.

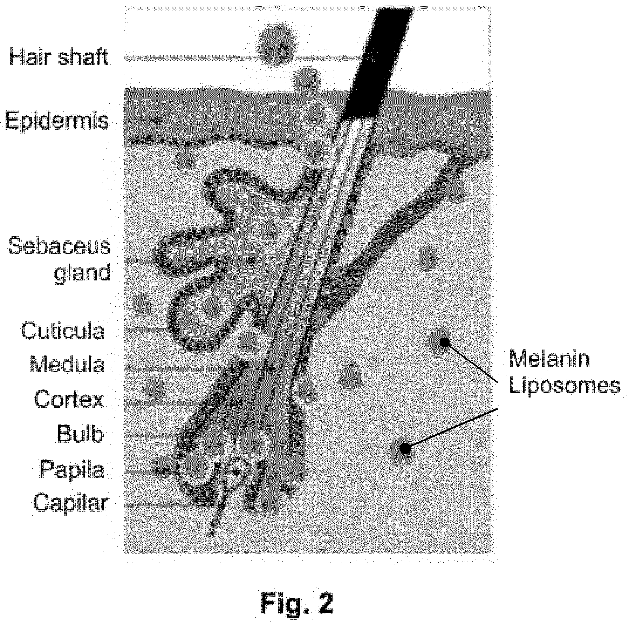

FIGURE 2. Shows a sectional view of the skin, in which its different components and those of the hair follicle are shown, in addition to a representation of the absorbed melanin liposomes.

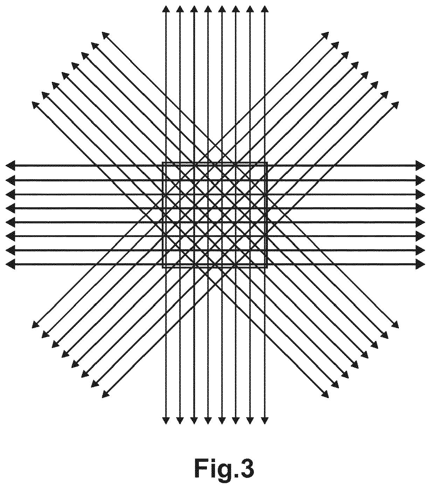

FIGURE 3. Shows a representation of the manner in which to apply the microneedle roller in order to achieve homogenous dermal puncture.

### PREFERRED EMBODIMENT OF THE INVENTION

In a preferred embodiment of the invention, the method is carried out using a liposomal sepia melanin solution, due to its resemblance to human melanin, as follows:
- Disinfection of the skin before applying the method.
- Shaving of the area of skin to be treated.
- Washing of the skin with cleansing lotion, rubbing a cotton bud impregnated with the lotion, without using alcohol or acetone.
- The melanin solution is applied at a concentration of 0.4 %-0.7 % onto the skin for 30 seconds, eight times in each direction, and gently massaged.
- Apply the melanin solution to the microneedle roller device. Penetration of the melanin solution through the skin is achieved by rolling the device having a roller or rollers over the area to be treated. The device has one or more small paint rollers, measuring 20 mm in diameter and 20 mm in length, and the cylinder surface houses 24 circular series of eight needles each, measuring 0.5 mm in length and 0.02 mm in diameter. The tool is made to roll for 3 minutes in different directions: horizontally, vertically and diagonally, and from right to left. This ensures an even perforation pattern, resulting in approximately 250-300 perforations/cm².
- The melanin solution is re-applied onto the treated area using the roller for 30 seconds, eight times in each direction, gently massaging for 1 or 2 minutes.
- The treated area is left to rest for a period of 5 to 7 minutes in order to allow full absorption of the melanin.
- Prior to laser exposure, the skin is washed using water and soap.
- Subject the skin to be treated to laser or IPL.

### CLINICAL STUDIES

Twenty patients were treated with microneedle rollers to produce a dermal puncture every 3 minutes combined with a liposomal melanin solution on one side of the arm and only with a melanin solution on the other side, and the result was evaluated.

**Results.** The side that received combined treatment (dermal puncture + melanin solution) showed a statistically significant reduction in comparison with the side treated only with a melanin solution and the clinical symptoms improved significantly.

**Conclusions.** This study suggests the potential use of combining dermal puncture with rollers and microneedles with a liposomal melanin solution to achieve better results in laser hair removal treatments for white, gray and blond hair.

### Histology: Two patients

Granular clusters of a brownish color in the stratum basale at intracellular level. These brownish-black deposits can be observed scattered throughout the papillary dermis. The post-laser biopsy reveals microvascular lesions (areas with extravasation of blood from capillaries) in the papillary dermis. Differences with regard to application with a roller and microneedles and without a roller can be observed. With a roller and microneedles the pigment is deeper and reaches the papillary dermis.

## Claims

1. An improved melanin-enhanced photothermolysis hair removal method for photoepilation of white, gray or blond hair, **characterized in that** prior to subjecting the skin to laser photoepilation, a device having rollers and microneedles is used in combination with the application of a melanin solution onto the area to be treated.

2. The improved melanin-enhanced photothermolysis hair removal method, according to claim 1, **characterized in that** it consists of the following stages:
- Disinfection of the skin prior to the method;
- Shaving of the area of skin to be treated;
- Cleaning of the skin without using alcohol or acetone;
- The melanin solution is applied onto the skin and gently massaged;
- Apply the melanin solution onto the device having rollers and microneedles, rolling it for 3 to 5 minutes over the area to be treated in different directions: horizontally, vertically and diagonally, and right and left;
- The melanin solution is re-applied onto the treated area and left to rest for a period of 7 to 8 minutes;
- Wash the skin using soap and water;
- Subject the skin to laser or IPL exposure.

3. The improved melanin-enhanced photothermolysis hair removal method, according to the preceding claims, **characterized in that** the concentration of melanin in the solution is 0.4 %-0.7 %.

4. The improved melanin-enhanced photothermolysis hair removal method, according to the preceding claims, **characterized in that** the optimum length of the roller microneedles is 0.5 mm.

## Patentansprüche

1. Verbessertes mit Melanin verstärktes Photothermolyse-Haarentfernungsverfahren zur Photoepilation von weißem, grauem oder blondem Haar, **dadurch gekennzeichnet, dass** bevor die Haut einer Laser-Photoepilation unterzogen wird, eine Vorrichtung mit Rollen und Mikronadeln zusammen mit der Auftragung einer Melaninlösung auf den zu behandelnden Bereich verwendet wird.

2. Verbessertes mit Melanin verstärktes Photothermolyse-Haarentfernungsverfahren nach Anspruch 1, **dadurch gekennzeichnet, dass** es aus den folgenden Schritten besteht:
Desinfizieren der Haut vor dem Verfahren;
- Rasieren des zu behandelnden Hautbereichs;
- Reinigen der Haut ohne Verwendung von Alkohol oder Aceton;
- Auftragen der Melaninlösung auf die Haut und sanftes Massieren;
- Auftragen der Melaninlösung auf die Vorrichtung mit Rollen und Mikronadeln, deren Rollen in verschiedene Richtungen: horizontal, vertikal und diagonal sowie nach rechts und links für 3 bis 5 Minuten über den zu behandelnden Bereich;
- Neuauftragen der Melaninlösung auf den zu behandelnden Bereich und Ruhenlassen für einen Zeitraum von 7 bis 8 Minuten;
- Waschen der Haut mit Wasser und Seife;
- Unterziehen der Haut einer Laser- oder IPL-Bestrahlung.

3. Verbessertes mit Melanin verstärktes Photothermolyse-Haarentfernungsverfahren nach den vorhergehenden Ansprüchen, **dadurch gekennzeichnet, dass** die Konzentration von Melanin in der Lösung 0,4 % bis 0,7 % beträgt.

4. Verbessertes mit Melanin verstärktes Photothermolyse-Haarentfernungsverfahren nach den vorhergehenden Ansprüchen, **dadurch gekennzeichnet, dass** die optimale Länge der Mikronadeln der Rolle 0,5 mm beträgt.

## Revendications

1. Procédé perfectionné d'épilation par photothermolyse améliorée avec la mélanine pour la photoépilation de poils blancs, gris ou blonds, **caractérisé en ce qu'**avant de soumettre la peau à la photoépilation au laser, un dispositif ayant des rouleaux et des microaiguilles est utilisé combiné à l'application d'une solution de mélanine sur la zone à traiter.

2. Procédé perfectionné d'épilation par photothermolyse améliorée avec la mélanine, selon la revendication 1, **caractérisé en ce qu'**il consiste en les étapes suivantes :
- Désinfection de la peau avant le procédé;
- Rasage de la zone de peau à traiter ;
- Nettoyage de la peau sans utiliser de l'alcool ou de l'acétone ;
- La solution de mélanine est appliquée sur la peau et doucement massée ;
- Appliquer la solution de mélanine sur le dispositif ayant des rouleaux et des microaiguilles, le faire rouler pendant 3 à 5 minutes sur la zone à traiter dans différentes directions : horizontalement, verticalement et diagonalement, et de gauche à droite ;
- La solution de mélanine est appliquée à nouveau sur la zone traitée et est laissée à reposer pour une période de 7 à 8 minutes :
- Laver la peau en utilisant du savon et de l'eau ;
- Soumettre la peau à une exposition laser ou IPL.

3. Procédé perfectionné d'épilation par photothermolyse améliorée avec la mélanine, selon les revendications précédentes, **caractérisé en ce que** la concentration de mélanine dans la solution est de 0,4 %-0,7 %.

4. Procédé perfectionné d'épilation par photothermolyse améliorée avec la mélanine, selon les revendications précédentes, **caractérisé en ce que** la longueur optimale des microaiguilles de rouleau est de 0,5 mm.
